# EUROPEAN PATENT APPLICATION

(11) **EP 4 848 330 A1**
(43) Date of publication of application: **02.12.2026**
(21) Application number: 25180039.7
(22) Date of filing: 30.05.2025
(51) Int. Cl.: H01J 49/00, G01N 33/68

(54) **APPARATUS AND METHOD**

(71) Applicant: Bruker Switzerland AG, 8117 Fällanden (CH)
(72) Inventor: PAPANASTASIOU, Dimitris, 8117 Fällanden (CH); ALEVIZOS, Georgios, 8117 Fällanden (CH); KOSMOPOULOU, Mariangela, 8117 Fällanden (CH); SUCKAU, Detlev, 8117 Fällanden (CH)
(74) Representative: Piticco, Lorena

(57) **Abstract**

A method of analyzing mass spectra, the method implemented by a computer including a processor and a memory, the method comprising:
obtaining a first mass spectrum comprising a first series of isotope peak clusters, including a first isotope peak cluster, corresponding to respective fragment ions of a first precursor ion for a first fragmentation mode of the first precursor ion, wherein the first precursor ion is of a first proteoform of a protein, a peptide or a part thereof, having one or more sequence variations, splice isoforms and/or post-translational modifications;
generating theoretical isotope peak clusters for the first series of isotope peak clusters for the first fragmentation mode of the first precursor ion; and
assigning the first series of isotope peak clusters for the first fragmentation mode of the first precursor ion using the generated theoretical isotope peak clusters;
wherein generating the theoretical isotope peak clusters for the first series of isotope peak clusters for the first fragmentation mode of the first precursor ion comprises creating, for example iteratively, a first set of candidate proteoforms of the protein, the peptide or the part thereof, having one or more sequence variations, splice isoforms and/or post-translational modifications, for example using an evolutionary algorithm, EA, such as a genetic algorithm, GA.

## Description

### FIELD

The technical field of the invention relates to methods of processing mass spectrometry data, for example mass spectra. For example, the invention may relate to methods of processing complex mass spectra generated by top-down mass spectrometry. For example, the invention may relate to methods of extracting sequence information from mass spectra of proteins and other classes of molecules *de novo.*

### BACKGROUND

Mass spectrometry (MS) is a powerful tool for analyzing proteins and other classes of biological macromolecules such as DNA/RNA and polysaccharides. Improvements in MS instrumentation offering enhanced sensitivity and mass resolving power have enabled the characterization of ions in the higher mass-to-charge (m/z) range. Top-down MS is concerned with the analysis of intact proteins subjected to gas phase fragmentation, yielding the molecular mass of the protein as well as protein ion fragments for deducing the primary structure of the protein. The level of information related to the primary structure of proteins has improved through the development of complementary dissociation methods, for example based on ion-electron and ion-photon interactions, leading to more complex and highly congested mass spectra containing a significantly increased number of fragments.

Despite the improvements in sample preparation, MS instrumentation and ion fragmentation methods, one of the most significant barriers for the proliferation of top-down MS workflows is the ability to analyze reliably the complex and highly congested mass spectra. In contrast to mainstream bottom-up MS where, for which a plethora of software tools has become available, software tools tailored to top-down MS data are lacking. The need for top-down MS software tools has become even more apparent in recent years since the efficiency of fragmentation methods, such as electron capture dissociation (ECD), electron induced dissociation (EID), ultraviolet photo-dissociation (UVPD), infrared multi-photon dissociation (IRMPD) and combinations thereof, has generated high quality but also remarkably complex top-down mass spectra. Hence, there is a clear need for improving the data analysis aspect of analytical workflows based on mass spectrometry.

Different approaches have been described to identify proteins based on tandem mass spectrometry (MS/MS) data. The most common approach is to utilize the available databases containing protein sequence information and/or to use existing libraries to match experimental MS/MS data. Spectral matching is typically applied for the identification of peptides that exhibit relatively simple fragment ion mass spectra where the monoisotopic mass of individual fragments can be accurately identified. In contrast, spectral matching becomes prone to errors as spectral complexity increases and monoisotopic peaks of higher m/z fragments are lost in the background. New methods are therefore necessary to measure the mass of the fragments reliably that consider the entire isotope clusters of the fragments and can further disentangle overlapping isotope distributions.

Moreover, the spectral matching approach suffers from several drawbacks and cannot be used for the identification of proteins from unknown genomes. Methods to directly extract the amino acid sequence information from the mass spectrum without the use of a database (*de novo* sequencing) have been developed. However, these are limited to peptides where spectra information is sparse. Sequencing heavily congested mass spectra *de novo* requires the development of new methods that account for the enhanced complexity due to extensive overlapping between isotope clusters. Furthermore, the excessive number of false positive *de novo* peptides from top down mass spectra must be reduced.

### SUMMARY OF THE INVENTION

A first aspect provides a method of analyzing mass spectra, the method implemented by a computer including a processor and a memory, the method comprising:
obtaining a first mass spectrum comprising a first series of isotope peak clusters, including a first isotope peak cluster, corresponding to respective fragment ions of a first precursor ion for a first fragmentation mode of the first precursor ion, wherein the first precursor ion is of a first proteoform of a protein, a peptide or a part thereof, having one or more sequence variations, splice isoforms and/or post-translational modifications;
generating theoretical isotope peak clusters for the first series of isotope peak clusters for the first fragmentation mode of the first precursor ion; and
assigning the first series of isotope peak clusters for the first fragmentation mode of the first precursor ion using the generated theoretical isotope peak clusters;
wherein generating the theoretical isotope peak clusters for the first series of isotope peak clusters for the first fragmentation mode of the first precursor ion comprises creating a first set of candidate proteoforms of the protein, the peptide or the part thereof, having one or more sequence variations, splice isoforms and/or post-translational modifications, for example using an evolutionary algorithm, EA, such as a genetic algorithm, GA.

A second aspect provides a method of identifying a proteoform of a protein, a peptide or a part thereof, having one or more sequence variations, splice isoforms and/or post-translational modifications, the method implemented by a computer including a processor and a memory, the method comprising:
analyzing a mass spectrum of the proteoform of the protein, the peptide or the part thereof, having one or more sequence variations, splice isoforms and/or post-translational modifications,
according to the first aspect; and
identifying the proteoform of the protein, the peptide or the part thereof, having one or more sequence variations, splice isoforms and/or post-translational modifications, using the first set of candidate proteoforms of the protein, the peptide or the part thereof, having one or more sequence variations, splice isoforms and/or post-translational modifications.

A third aspect provides a method of analyzing mass spectra, the method implemented by a computer including a processor and a memory, the method comprising:
obtaining a first mass spectrum comprising a first series of isotope peak clusters, including a first isotope peak cluster, corresponding to respective fragment ions of a first precursor ion for a first fragmentation mode of the first precursor ion, herein the first precursor ion is of a molecule having one or more variations and/or modifications;
generating theoretical isotope peak clusters for the first series of isotope peak clusters for the first fragmentation mode of the first precursor ion; and
assigning the first series of isotope peak clusters for the first fragmentation mode of the first precursor ion using the generated theoretical isotope peak clusters;
wherein generating the theoretical isotope peak clusters for the first series of isotope peak clusters for the first fragmentation mode of the first precursor ion comprises creating a first set of candidate forms of the molecule, having one or more variations and/or modifications, for example using an evolutionary algorithm, EA, such as a genetic algorithm, GA.

A fourth aspect provides a method of identifying a molecule having one or more variations and/or modifications, the method implemented by a computer including a processor and a memory, the method comprising:
analyzing a mass spectrum of the molecule having one or more variations and/or modifications, according to the third aspect; and
identifying the molecule having one or more variations and/or modifications, using the first set of candidate proteoforms of the molecule having one or more variations and/or modifications.

A fifth aspect provides: a computer including a processor and a memory configured to perform a method according to any one or more of the first aspect to the fourth aspect; a computer program comprising instructions which, when executed by a computer including a processor and a memory, cause the computer to perform a method according to any one or more of the first aspect to the fourth aspect; and/or a non-transient computer-readable storage medium comprising instructions which, when executed by a computer including a processor and a memory, cause the computer to perform a method according to any one or more of the first aspect to the fourth aspect.

A sixth aspect provides a mass spectrometer comprising a computer including a processor and a memory configured to perform a method according to any one or more of the first aspect to the fourth aspect.

### DETAILED DESCRIPTION OF THE INVENTION

### First aspect

The first aspect provides a method of analyzing mass spectra, the method implemented by a computer including a processor and a memory, the method comprising:
obtaining a first mass spectrum comprising a first series of isotope peak clusters, including a first isotope peak cluster, corresponding to respective fragment ions of a first precursor ion for a first fragmentation mode of the first precursor ion, wherein the first precursor ion is of a first proteoform of a protein, a peptide or a part thereof, having one or more sequence variations, splice isoforms and/or post-translational modifications;
generating theoretical isotope peak clusters for the first series of isotope peak clusters for the first fragmentation mode of the first precursor ion; and
assigning the first series of isotope peak clusters for the first fragmentation mode of the first precursor ion using the generated theoretical isotope peak clusters;
wherein generating the theoretical isotope peak clusters for the first series of isotope peak clusters for the first fragmentation mode of the first precursor ion comprises creating, for example iteratively, a first set of candidate proteoforms of the protein, the peptide or the part thereof, having one or more sequence variations, splice isoforms and/or post-translational modifications, for example using an evolutionary algorithm, EA, such as a genetic algorithm, GA.

In this way, identification of the first proteoform of the protein is improved, for example by improving assignment of the first series of isotope clusters, such as by increasing confidence of the assignment (more true positives, fewer false positives, more true negatives and/or fewer false negatives), since the assignment uses the created first set of candidate proteoforms of the protein, the peptide or the part thereof. In this way, identification of the first proteoform of the protein is improved, for example by improving sequence coverage. In this way, a speed of identification of the first proteoform of the protein may be improved, for example using an EA such as a GA, which may rapidly converge towards a candidate proteoform that is consistent with and/or corresponds with the first proteoform.

In this way, given the first mass spectrum and a sequence of amino acid residues, where each of the amino acid residues may be variably modified by one or more post-translational modifications (PTMs), for example, the method finds the most probable configuration of PTMs (i.e. candidate proteoform) such that the theoretical fragments of that configuration of PTMs best explain the first mass spectrum.

A **variable modification** expresses our uncertainty as to whether a specific PTM is either present or not on a specific amino acid residue. Adding a variable modification to an unmodified amino acid residue in the sequence introduces two possible states. One in which the modification is present and the other in which it is not. For any additional variable modifications on this amino acid, an extra state is added. So, if **Ni** is the number of variable modifications on amino acid residue i of the input sequence, applying a variable modification on the amino acid **j** results in **(Ni + 1) * (Nj + 1)** configurations due to the combination of all states. Since this is a product, applying even more variable modifications to different amino acid residues significantly increases the number of configurations, e.g., having defined a variable modification on thirty-two different amino acid residues will result in 2^32 (approximately 4.3 billion) possible states. With that in mind, evaluating each one of these states individually (especially for more than 15-20 variably modified sites) poses a potentially computationally infeasible problem.

The method of is of analyzing mass spectra, for example MS/MS or MS² mass spectra, more generally MSⁿ mass spectra wherein n is a natural number greater than or equal to 2, for example 2, 3, 4, 5. It should be understood that the mass spectra are of proteins, peptides or parts thereof, generally comprising a plurality of amino acids. It should be understood that more generally, the method may additionally and/or alternatively be used for analyzing mass spectra of other complex molecules, such as oligonucleotides and peptide-oligonucleotide conjugates mutatis mutandis.

The method is implemented by the computer including the processor and the memory. Suitable computers are known.

The method comprises obtaining the first mass spectrum comprising the first series of isotope peak clusters, including the first isotope peak cluster, corresponding to respective fragment (also known as product) ions of the first precursor ion for the first fragmentation mode of the first precursor ion. It should be understood that the first mass spectrum comprises and/or is a MS/MS or MS² mass spectrum, more generally MSⁿ mass spectrum wherein n is a natural number greater than or equal to 2, for example 2, 3, 4, 5. It should be understood that an isotope peak cluster comprises a set of mass spectral peaks corresponding with respective isotope combinations for an ion and which may be characterized by the monoisotopic peak thereof. It should be understood that respective mass spectra for a protein, a peptide or a part thereof and for proteoform of a protein, a peptide or a part thereof typically comprise a plurality of mutually-overlapping isotope peak clusters, that may not be resolved based on m/z. It should be understood that more generally, the method may additionally and/or alternatively be used for nth generation ions and n+1th generation fragment ions thereof. In one example, the first fragmentation (also known as dissociation) mode is selected from a list consisting of: CID, SID, ETD, NETD, PD, ECD, hECD, EED, EID, El, niECD, EDD; preferably ECD, hECD, EED, EID, El, niECD, EDD. Fragmentation modes include Electron Capture Dissociation (ECD), hot Electron Capture Dissociation (hECD), Electron Excitation Dissociation (EED), Electron Energy Dissociation (EID), Electron Impact (El), negative ion ECD (niECD) and Electron Detachment Dissociation (EDD). Fragmentation modes also include collision-induced dissociation (CID) through collision with neutral molecule, surface-induced dissociation (SID) using fast moving ions collision with a solid surface, laser induced dissociation which uses laser to induce the ion formation, electron-capture dissociation (ECD) due to capturing of low energy electrons, electron-transfer dissociation (ETD) through electron transfer between ions, negative electron-transfer dissociation (NETD), electron-detachment dissociation (EDD), photodissociation (PD), particularly infrared multiphoton dissociation (IRMPD) using IR radiation for the bombardment and blackbody infrared radiative dissociation (BIRD) which use IR radiation instead of laser, higher-energy C-trap dissociation (HCD), EISA, and charge remote fragmentation.

In one example, obtaining the first mass spectrum comprises denoising, peak picking and/or deconvolution, for example using a deconvolution algorithm as described in PCT/EP2025/064439, the subject matter of which is incorporated herein in entirety.

The first precursor ion is of the first proteoform of a protein, a peptide or a part thereof, having one or more sequence variations, splice isoforms and/or post-translational modifications. Generally, a proteoform is a particular molecular form of a protein, having one or more sequence variations, splice isoforms and/or post-translational modifications, and herein more generally includes also a peptide or a part thereof, having one or more sequence variations, splice isoforms and/or post-translational modifications.

The method comprises generating theoretical isotope peak clusters for the first series of isotope peak clusters for the first fragmentation mode of the first precursor ion. In this way, theoretical isotope peak clusters corresponding with the first series of isotope peak clusters are generated, thereby enabling assignment thereof.

The method comprises assigning the first series of isotope peak clusters for the first fragmentation mode of the first precursor ion using the generated theoretical isotope peak clusters. In this way, the first series of isotope peak clusters are assigned to the generated theoretical isotope peak clusters (and/or vice versa), thereby enabling identification of the first proteoform of a protein, a peptide or a part thereof, having one or more sequence variations, splice isoforms and/or post-translational modifications.

Generating the theoretical isotope peak clusters for the first series of isotope peak clusters for the first fragmentation mode of the first precursor ion comprises creating, for example iteratively, a first set of candidate proteoforms of the protein, the peptide or the part thereof, having one or more sequence variations, splice isoforms and/or post-translational modifications. In this way, candidate proteoforms are created and respective theoretical isotope peak clusters generated therefrom, for assignment to the first series of isotope peak clusters.

By creating iteratively the first set of candidate proteoforms, further candidate proteoforms may be created based on previously created candidate proteoforms, thereby improving assignment thereof.

In one example, creating iteratively the first set of candidate proteoforms of the protein, the peptide or the part thereof, having one or more sequence variations, splice isoforms and/or post-translational modifications comprises using an evolutionary algorithm, EA, such as a genetic algorithm, GA. Generally, a GA is a metaheuristic optimization algorithm inspired by the process of natural selection that belongs to the larger class of EAs. A GA iteratively refines a population of candidate solutions (individuals) to find the best possible solution to a given problem via biologically inspired operators such as selection, crossover, and mutation.

In one example, creating the first set of candidate proteoforms of the protein, the peptide or the part thereof comprises creating an initial first set of candidate proteoforms of the protein, the peptide or the part thereof (for example, an initial population of candidate proteoforms), for example by systematically, combinatorially and/or randomly proposing one or more sequence variations, splice isoforms and/or post-translational modifications to the protein, the peptide or the part thereof. In this way, an initial population of candidate proteoforms may be created, including systematically, combinatorially and/or randomly one or more sequence variations, splice isoforms and/or post-translational modifications of the protein, the peptide or the part thereof.

In one example, creating the first set of candidate proteoforms of the protein, the peptide or the part thereof comprises proposing, for example repeatedly, one or more sequence variations, splice isoforms and/or post-translational modifications of the protein, the peptide or the part thereof. In this way, an initial population and/or refined populations (i.e. of the initial population and/or a previous refined population) of candidate proteoforms may be created.

In one example, creating the first set of candidate proteoforms of the protein, the peptide or the part thereof comprises proposing, for example successively, one or more sequence variations, splice isoforms and/or post-translational modifications of a candidate proteoform of the protein, the peptide or the part thereof. In this way, refined populations (i.e. of the initial population and/or a previous refined population) of candidate proteoforms may be created.

In one example, creating the first set of candidate proteoforms of the protein, the peptide or the part thereof comprises including, for example including only, selected candidate proteoforms of the protein, the peptide or the part thereof, for example having respective rankings of at least a predetermined rank, for example relatively or absolutely. In this way, selected candidate proteoforms are retained in the first set of candidate proteoforms. In this way, the first set of proteoforms is optimized by including, for example only, the selected candidate proteoforms, for example having respective rankings of at least a predetermined rank, for example relatively or absolutely. For example, only selected candidate proteoforms having respective rankings of at least the predetermined rank may be retained.

In one example, creating the first set of candidate proteoforms of the protein, the peptide or the part thereof comprises excluding, for example excluding only, selected candidate proteoforms of the protein, the peptide or the part thereof, for example having respective rankings of less than a predetermined rank, for example relatively or absolutely. In this way, selected candidate proteoforms are removed from the first set of candidate proteoforms. In this way, the first set of proteoforms is optimized by excluding, for example only, the selected candidate proteoforms, for example having respective rankings of less than the predetermined rank, for example relatively or absolutely. For example, only selected candidate proteoforms having respective rankings of less than the predetermined rank may be removed.

In one example, creating the first set of candidate proteoforms of the protein, the peptide or the part thereof comprises iteratively creating the first set of candidate proteoforms of the protein, the peptide or the part thereof, for example until a predetermined convergence criterion is met. In this way, the first set of proteoforms is iteratively optimized, for example until a predetermined convergence criterion is met. In one example, the predetermined convergence criterion comprises and/or is a predetermined scoring and/or a predetermined number of iterations.

In one example, generating the theoretical isotope peak clusters for the first series of isotope peak clusters for the first fragmentation mode of the first precursor ion comprises generating respective theoretical isotope peak clusters of respective fragments for the first fragmentation mode of the respective candidate proteoforms of the first set of candidate proteoforms of the protein, the peptide or the part thereof. In this way, the respective theoretical isotope peak clusters of respective fragments for the first fragmentation mode of the respective candidate proteoforms are generated, for example for assignment to the first series of isotope peak clusters (or vice versa).

In one example, creating the first set of candidate proteoforms of the protein, the peptide or the part thereof comprises ranking respective candidate proteoforms of the first set of candidate proteoforms of the protein, the peptide or the part thereof. In this way, selected candidate proteoforms may be included or excluded, as described previously.

In one example, ranking respective candidate proteoforms of the first set of candidate proteoforms of the protein, the peptide or the part thereof comprises ranking respective candidate proteoforms of the first set of candidate proteoforms of the protein, the peptide or the part thereof based on respective results of scoring respective theoretical isotope peak clusters. In this way, the candidate proteoforms may be quantitatively ranked based on respective scorings.

In one example, scoring respective theoretical isotope peak clusters comprises scoring respective theoretical isotope peak clusters based on respective results of fitting respective theoretical isotope peak clusters to the first series of isotope peak clusters. In this way, candidate proteoforms may be quantitatively ranked based on respective scorings of fitting respective theoretical isotope peak clusters to the first series of isotope peak clusters, for example by matching.

In one example, scoring respective theoretical isotope peak clusters comprises scoring respective theoretical isotope peak clusters based on respective results of estimating respective sequence coverages of the respective candidate proteoforms of the first set of candidate proteoforms of the protein, the peptide or the part thereof. In this way, candidate proteoforms may be quantitatively ranked based on respective scorings of sequence coverage.

In one example, the first mass spectrum comprises a second series of isotope peak clusters, including a first isotope peak cluster, corresponding to respective fragment ions of a second precursor ion for a first fragmentation mode of the second precursor ion, wherein the second precursor ion is of a second proteoform of the protein, the peptide or the part thereof, having one or more sequence variations, splice isoforms and/or post-translational modifications. In this way, positional isomers of the proteoform may be distinguished. For example, positional isomers of the proteoform may be co-isolated in a fragmentation device, resulting in chimeric MS/MS spectra. In this way, such positional isomers may be identified, for example individually, since they result in different fragments. The second series of isotope peak clusters, the second precursor ion and/or the second proteoform may be as described respectively with respect to the first series of isotope peak clusters, the first precursor ion and/or the first proteoform mutatis mutandis.

In one example, the method comprises obtaining a second mass spectrum comprising a first series of isotope peak clusters, including a first isotope peak cluster, corresponding to respective fragment ions of the first precursor ion for a second fragmentation mode of the first precursor ion; generating theoretical isotope peak clusters for the first series of isotope peak clusters for the second fragmentation mode of the first precursor ion; and
assigning the first series of isotope peak clusters for the second fragmentation mode of the first precursor ion using the generated theoretical isotope peak clusters;
wherein generating the theoretical isotope peak clusters for the first series of isotope peak clusters for the second fragmentation mode of the first precursor ion comprises creating, for example iteratively, a second set of candidate proteoforms of the protein, the peptide or the part thereof, having one or more sequence variations, splice isoforms and/or post-translational modifications, for example using an evolutionary algorithm, EA, such as a genetic algorithm, GA.

In this way, identification of the proteoform may be confirmed using a second fragmentation mode. The second mass spectrum and/or the second fragmentation mode may be as described respectively with respect to the first mass spectrum and/or the first fragmentation mode mutatis mutandis. It should be understood that the first fragmentation mode and the second fragmentation mode are mutually different.

In one example, the method comprises comparing the first set of candidate proteoforms of the protein, the peptide or the part thereof and the second set of candidate proteoforms of the protein, the peptide or the part thereof. In this way, identification of the proteoform may be confirmed using a second fragmentation mode, by comparing the first set of candidate proteoforms of the protein and the second set of candidate proteoforms.

In one example, the method comprises choosing a candidate proteoform of the protein, the peptide or the part thereof based on a result of the comparing. In this way, identification of the proteoform may be confirmed using a second fragmentation mode, by comparing the first set of candidate proteoforms of the protein and the second set of candidate proteoforms.

### Second aspect

The second aspect provides a method of identifying a proteoform of a protein, a peptide or a part thereof, having one or more sequence variations, splice isoforms and/or post-translational modifications, the method implemented by a computer including a processor and a memory, the method comprising:
analyzing a mass spectrum of the proteoform of the protein, the peptide or the part thereof, having one or more sequence variations, splice isoforms and/or post-translational modifications, according to the first aspect; and
identifying the proteoform of the protein, the peptide or the part thereof, having one or more sequence variations, splice isoforms and/or post-translational modifications, using the first set of candidate proteoforms of the protein, the peptide or the part thereof, having one or more sequence variations, splice isoforms and/or post-translational modifications.

### Third aspect

The third aspect provides a method of analyzing mass spectra, the method implemented by a computer including a processor and a memory, the method comprising:
obtaining a first mass spectrum comprising a first series of isotope peak clusters, including a first isotope peak cluster, corresponding to respective fragment ions of a first precursor ion for a first fragmentation mode of the first precursor ion, herein the first precursor ion is of a molecule having one or more variations and/or modifications;
generating theoretical isotope peak clusters for the first series of isotope peak clusters for the first fragmentation mode of the first precursor ion; and
assigning the first series of isotope peak clusters for the first fragmentation mode of the first precursor ion using the generated theoretical isotope peak clusters;
wherein generating the theoretical isotope peak clusters for the first series of isotope peak clusters for the first fragmentation mode of the first precursor ion comprises creating a first set of candidate forms of the molecule, having one or more variations and/or modifications, for example using an evolutionary algorithm, EA, such as a genetic algorithm, GA.

The method according to the third aspect may be generally as described with respect to the method according to the first aspect mutatis mutandis.

### Fourth aspect

The fourth aspect provides a method of identifying a molecule having one or more variations and/or modifications, the method implemented by a computer including a processor and a memory, the method comprising:
analyzing a mass spectrum of the molecule having one or more variations and/or modifications, according to the third aspect; and
identifying the molecule having one or more variations and/or modifications, using the first set of candidate proteoforms of the molecule having one or more variations and/or modifications.

The method according to the fourth aspect may be generally as described with respect to the method according to the second aspect mutatis mutandis.

### Fifth aspect

The fifth aspect provides: a computer including a processor and a memory configured to perform a method according to any one or more of the first aspect to the fourth aspect; a computer program comprising instructions which, when executed by a computer including a processor and a memory, cause the computer to perform a method according to any one or more of the first aspect to the fourth aspect; and/or a non-transient computer-readable storage medium comprising instructions which, when executed by a computer including a processor and a memory, cause the computer to perform a method according to any one or more of the first aspect to the fourth aspect.

### Sixth aspect

The sixth aspect provides a mass spectrometer comprising a computer including a processor and a memory configured to perform a method according to any one or more of the first aspect to the fourth aspect.

### BRIEF DESCRIPTION OF THE DRAWINGS

For a better understanding of the invention, and to show how exemplary embodiments of the same may be brought into effect, reference will be made, by way of example only, to the accompanying diagrammatic Figures, in which:
Figure 1 schematically depicts a method according to an exemplary embodiment;
Figure 2A and Figure 2B schematically depict a method according to an exemplary embodiment;
Figure 3 shows a MS2 ECD mass spectrum of AMPK β1, for Top Down Characterization of AMP Kinase by FTICR-MS;
Figure 4 shows a method according to an exemplary embodiment wherein PTM screening is applied to 34 billion possible proteoforms of AMPK β1, identifying the most abundant proteoform for the mass spectrum of Figure 3; and
Figure 5 shows the corresponding sequence map for the method of Figure 4.

### DETAILED DESCRIPTION OF THE DRAWINGS

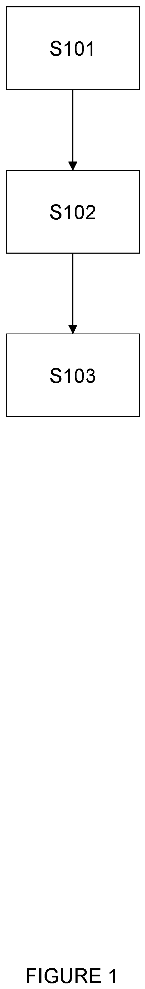

Figure 1 schematically depicts a method according to an exemplary embodiment.

The method is of analyzing mass spectra and the method is implemented by a computer including a processor and a memory.

The method comprises:
obtaining a first mass spectrum comprising a first series of isotope peak clusters, including a first isotope peak cluster, corresponding to respective fragment ions of a first precursor ion for a first fragmentation mode of the first precursor ion, wherein the first precursor ion is of a first proteoform of a protein, a peptide or a part thereof, having one or more sequence variations, splice isoforms and/or post-translational modifications (S101);
generating theoretical isotope peak clusters for the first series of isotope peak clusters for the first fragmentation mode of the first precursor ion (S102); and
assigning the first series of isotope peak clusters for the first fragmentation mode of the first precursor ion using the generated theoretical isotope peak clusters (S103);
wherein generating the theoretical isotope peak clusters for the first series of isotope peak clusters for the first fragmentation mode of the first precursor ion comprises creating, for example iteratively, a first set of candidate proteoforms of the protein, the peptide or the part thereof, having one or more sequence variations, splice isoforms and/or post-translational modifications, for example using an evolutionary algorithm, EA, such as a genetic algorithm, GA.

The method may include any step described with respect to the first aspect.

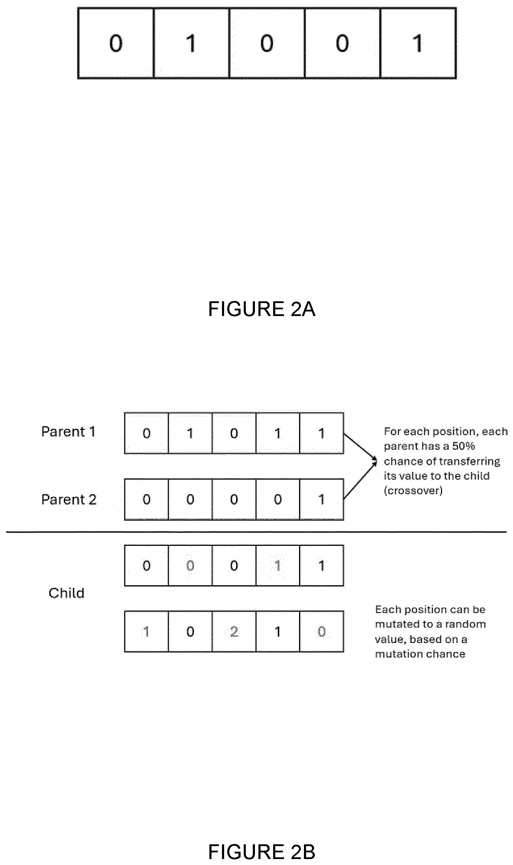

Figure 2A and Figure 2B schematically depict a method according to an exemplary embodiment.

Before explaining how exploring the whole space of possible evaluations can be bypassed, it is useful to specify how each evaluation is being performed.

Initially, the input spectrum undergoes denoising, peak picking and deconvolution, for example using a deconvolution algorithm. The result of this sequence of operations is a list of isotopic distributions in the neutral mass domain corresponding to different experimental fragments. In practice, any deconvolution algorithm that provides a comparable output can be used.

For each proteoform (a sequence with a specific combination of PTMs) to be evaluated, theoretical fragments are generated based on the fragmentation mode of the experiment. The next step is to try and match the monoisotopic mass of each one of these theoretical fragments to any monoisotopic mass in the list of the deconvoluted isotopic distributions, within a user specified mass tolerance.

Any successful match contributes to the total score of each evaluation based on two different metrics, the sequence coverage and the intensity coverage, i.e., the intensity of all matching fragments divided by the sum of all intensities of the deconvoluted isotopic distributions).

To efficiently explore the space of possible combinations, we utilize Genetic Algorithms (GA). Genetic Algorithms are advanced optimization techniques inspired by the principles of natural selection and belong to the broader category of Evolutionary Algorithms (EA).

### Method

In our scenario, we utilize a GA in the following way:
1) Generate an initial population of N possible solutions by randomly selecting available PTMs on the sequence of each child solution. We essentially flip a coin on every amino acid that has a variable modification in each sequence and based on the outcome, we either include the PTM or not.
   Each member of the population has a list of integer elements indicating which PTM out of the available ones in that position is selected. The 0 element is used to indicate the absence of a PTM in that position. For example, if the input sequence is 5 amino acids long, this list may be shown by Figure 2A, in which the number 1 indicates that PTM 1 is present on positions 2 and 5 and the number 0 indicates that a PTM is absent on positions 1, 3 and 4.
2) Evaluate each configuration (proteoform) and sort the solutions from best to worst, based on their total scores.
3) Select a percentage of the top solutions as parents for the next generation and randomly assign N pairs of these parents for them to produce child solutions (Figure 2B).
4) Each child solution is generated. The mutation chance parameter is extremely important to any genetic algorithm, as mutation is the driving force in exploring various solutions and not staying dependent on the solutions that were initially randomly generated. A very high chance of mutations can result in exploring many different combinations of solutions with the danger of poorly converging on a potentially good one, whereas a very low chance will potentially cause the opposite effect, sacrificing exploration of the solution space for a potential exploitation of good solutions. For our use cases, a mutation rate from 10% to 20% is usually suitable.
5) The children of the parents of the previous generation now constitute the new parent population.
6) Repeat steps 2 - 5 until convergence or reaching an upper limit on the number of generations or running time. Example convergence criteria can include an unimproved top solution for a consecutive number of generations. (The algorithm will not stop until the top solution has not been improved for a specific number of generations).

### Result

As each generation progresses, we maintain a record of the top K configurations identified. This record represents the output of the algorithm. With a properly calibrated Genetic Algorithm (GA), multiple executions with identical input typically converge to the same top K configurations, provided that the fragment ions in the input spectrum are sufficiently distinct to differentiate between various optimal solutions.

### RESULTS

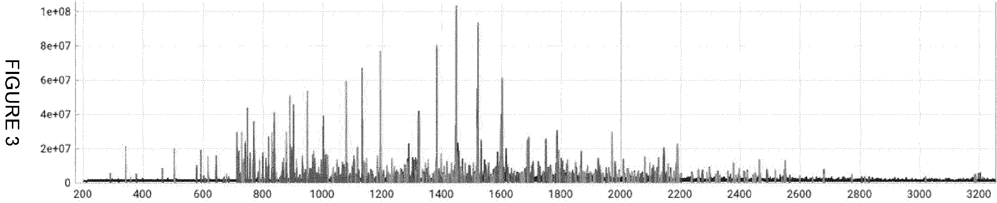

Figure 3 shows a MS2 ECD mass spectrum of AMPK β1, for Top Down Characterization of AMP Kinase by FTICR-MS.

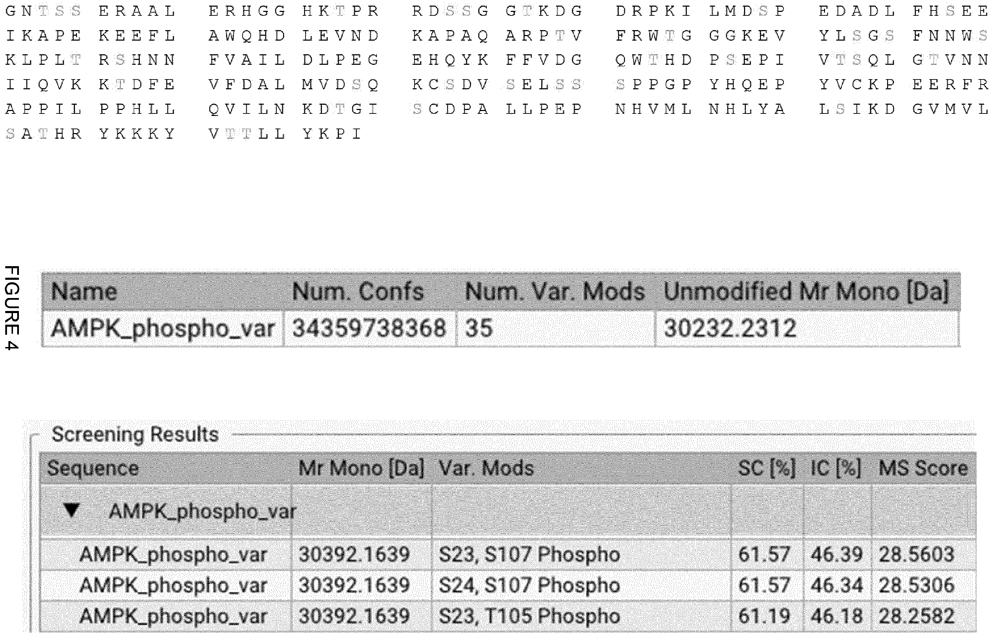

Figure 4 shows a method according to an exemplary embodiment wherein PTM screening is applied to 34 billion possible proteoforms of AMPK β1, identifying the most abundant proteoform for the mass spectrum of Figure 3.

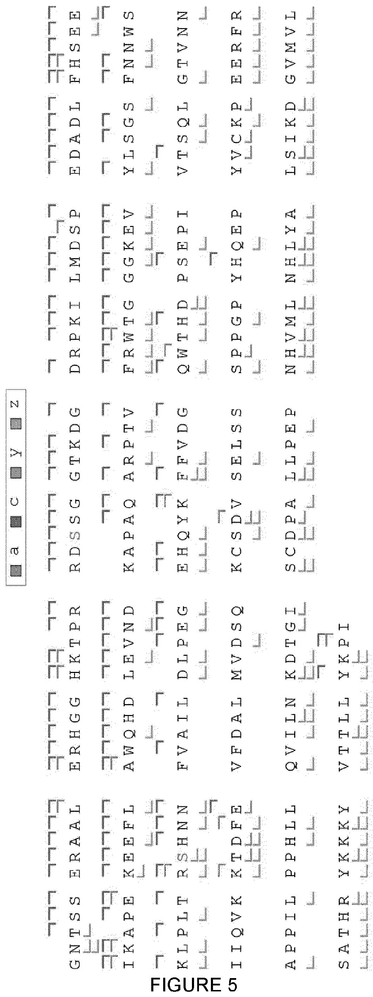

Figure 5 shows the corresponding sequence map for the method of Figure 4.

PTM screening is based on the total number of possible proteoforms for a given protein family. A brute force method is applied for a smaller search space, while a heuristic approach is adopted for an extreme number of possible protein configurations of 2n, even with n>100, achieving convergence within a few seconds.

The new features of the software are evaluated using TD mass spectra of AMPK-β and carbonic anhydrase II (CA II), using ScimaX MRMS and maXis II ETD instruments (Bruker), respectively.

The PTM screening workflow is optimized to process an extremely high number of protein configurations and identify the most probable one, allowing screening of billions of proteoforms within seconds. In this example, AMPK has 35 possible modifications on Serines and Threonines, corresponding to 34e9 possible protein configurations

The heuristic method identifies the correct proteoform of the AMPK subunit with 2 phosphorylation sites (S23, S107) in just a few seconds.

The PTM Screening also provides an effective method for PTM identification when co-isolated ions undergo fragmentation. This scenario can arise with co-isolated positional isomers or chimeric MS/MS spectra, which are generated due to the quadrupole mass filter's limited resolving capabilities.

### NOTES

Although a preferred embodiment has been shown and described, it will be appreciated by those skilled in the art that various changes and modifications might be made without departing from the scope of the invention, as defined in the appended claims and as described above.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A method of analyzing mass spectra, the method implemented by a computer including a processor and a memory, the method comprising:
obtaining a first mass spectrum comprising a first series of isotope peak clusters, including a first isotope peak cluster, corresponding to respective fragment ions of a first precursor ion for a first fragmentation mode of the first precursor ion, wherein the first precursor ion is of a first proteoform of a protein, a peptide or a part thereof, having one or more sequence variations, splice isoforms and/or post-translational modifications;
generating theoretical isotope peak clusters for the first series of isotope peak clusters for the first fragmentation mode of the first precursor ion; and
assigning the first series of isotope peak clusters for the first fragmentation mode of the first precursor ion using the generated theoretical isotope peak clusters;
wherein generating the theoretical isotope peak clusters for the first series of isotope peak clusters for the first fragmentation mode of the first precursor ion comprises creating, for example iteratively,
a first set of candidate proteoforms of the protein, the peptide or the part thereof, having one or more sequence variations, splice isoforms and/or post-translational modifications, for example using an evolutionary algorithm, EA, such as a genetic algorithm, GA.

2. The method according to any previous claim, wherein creating the first set of candidate proteoforms of the protein, the peptide or the part thereof comprises proposing, for example repeatedly, one or more sequence variations, splice isoforms and/or post-translational modifications of the protein, the peptide or the part thereof.

3. The method according to any previous claim, wherein creating the first set of candidate proteoforms of the protein, the peptide or the part thereof comprises proposing, for example successively, one or more sequence variations, splice isoforms and/or post-translational modifications of a candidate proteoform of the protein, the peptide or the part thereof.

4. The method according to any previous claim, wherein creating the first set of candidate proteoforms of the protein, the peptide or the part thereof comprises including, for example including only, selected candidate proteoforms of the protein, the peptide or the part thereof, for example having respective rankings of at least a predetermined rank.

5. The method according to any previous claim, wherein creating the first set of candidate proteoforms of the protein, the peptide or the part thereof comprises excluding, for example excluding only, selected candidate proteoforms of the protein, the peptide or the part thereof, for example having respective rankings of less than a predetermined rank.

6. The method according to any previous claim, wherein creating the first set of candidate proteoforms of the protein, the peptide or the part thereof comprises iteratively creating the first set of candidate proteoforms of the protein, the peptide or the part thereof, for example until a predetermined convergence criterion is met.

7. The method according to any previous claim, wherein generating the theoretical isotope peak clusters for the first series of isotope peak clusters for the first fragmentation mode of the first precursor ion comprises generating respective theoretical isotope peak clusters of respective fragments for the first fragmentation mode of the respective candidate proteoforms of the first set of candidate proteoforms of the protein, the peptide or the part thereof.

8. The method according to any previous claim, wherein creating the first set of candidate proteoforms of the protein, the peptide or the part thereof comprises ranking respective candidate proteoforms of the first set of candidate proteoforms of the protein, the peptide or the part thereof.

9. The method according to claim 8, wherein ranking respective candidate proteoforms of the first set of candidate proteoforms of the protein, the peptide or the part thereof comprises ranking respective candidate proteoforms of the first set of candidate proteoforms of the protein, the peptide or the part thereof based on respective results of scoring respective theoretical isotope peak clusters.

10. The method according to claim 9, wherein scoring respective theoretical isotope peak clusters comprises scoring respective theoretical isotope peak clusters based on respective results of fitting respective theoretical isotope peak clusters to the first series of isotope peak clusters.

11. The method according to any of claims 9 to 10, wherein scoring respective theoretical isotope peak clusters comprises scoring respective theoretical isotope peak clusters based on respective results of estimating respective sequence coverages of the respective candidate proteoforms of the first set of candidate proteoforms of the protein, the peptide or the part thereof.

12. The method according to any previous claim, wherein the first mass spectrum comprises a second series of isotope peak clusters, including a first isotope peak cluster, corresponding to respective fragment ions of a second precursor ion for a first fragmentation mode of the second precursor ion, wherein the second precursor ion is of a second proteoform of the protein, the peptide or the part thereof, having one or more sequence variations, splice isoforms and/or post-translational modifications.

13. The method according to any previous claim, comprising obtaining a second mass spectrum comprising a first series of isotope peak clusters, including a first isotope peak cluster, corresponding to respective fragment ions of the first precursor ion for a second fragmentation mode of the first precursor ion;
generating theoretical isotope peak clusters for the first series of isotope peak clusters for the second fragmentation mode of the first precursor ion; and
assigning the first series of isotope peak clusters for the second fragmentation mode of the first precursor ion using the generated theoretical isotope peak clusters;
wherein generating the theoretical isotope peak clusters for the first series of isotope peak clusters for the second fragmentation mode of the first precursor ion comprises creating, for example iteratively, a second set of candidate proteoforms of the protein, the peptide or the part thereof, having one or more sequence variations, splice isoforms and/or post-translational modifications, for example using an evolutionary algorithm, EA, such as a genetic algorithm, GA.

14. The method according to claim 13, comprising comparing the first set of candidate proteoforms of the protein, the peptide or the part thereof and the second set of candidate proteoforms of the protein, the peptide or the part thereof; and optionally choosing a candidate proteoform of the protein, the peptide or the part thereof based on a result of the comparing.

15. A method of identifying a proteoform of a protein, a peptide or a part thereof, having one or more sequence variations, splice isoforms and/or post-translational modifications, the method implemented by a computer including a processor and a memory, the method comprising:
analyzing a mass spectrum of the proteoform of the protein, the peptide or the part thereof, having one or more sequence variations, splice isoforms and/or post-translational modifications, according to any previous claim; and
identifying the proteoform of the protein, the peptide or the part thereof, having one or more sequence variations, splice isoforms and/or post-translational modifications, using the first set of candidate proteoforms of the protein, the peptide or the part thereof, having one or more sequence variations, splice isoforms and/or post-translational modifications.
